(19) Europäisches Patentamt
European Patent Office
Office européen des brevets

(11) EP 0 647 217 B1

# (12) EUROPEAN PATENT SPECIFICATION

(45) Date of publication and mention of the grant of the patent: **29.10.1997 Bulletin 1997/44**

(21) Application number: **93913223.9**

(22) Date of filing: **28.05.1993**

(51) Int Cl.[6]: **C07C 237/42**, C07C 259/10, A01N 37/42, A01N 37/52

(86) International application number: **PCT/GB93/01115**

(87) International publication number: **WO 94/00422 (06.01.1994 Gazette 1994/02)**

## (54) 4-ACYLAMINOBENZAMIDES AND THEIR USE AS FUNGICIDES

4-ACYLAMINO-BENZAMIDE UND DEREN VERWENDUNG ALS FUNGIZIDE

4-ACYLAMINOBENZAMIDES ET LEUR UTILISATION COMME FONGICIDES

(84) Designated Contracting States:
**BE CH DE DK ES FR GB IE IT LI NL PT**

(30) Priority: **26.06.1992 GB 9213568**
**13.08.1992 GB 9217122**
**13.08.1992 GB 9217126**

(43) Date of publication of application:
**12.04.1995 Bulletin 1995/15**

(73) Proprietor: **ZENECA LIMITED**
**London W1Y 6LN (GB)**

(72) Inventors:
- **CROWLEY, Jelf, Patrick 56 Ellis Road Berkshire RG11 6PT (GB)**
- **LAWSON, Kevin, Robert High Stile Piddington Lane High Wycombe Buckinghamshire HP14 3BB (GB)**

(74) Representative: **Tierney, Francis John et al**
**Intellectual Property Department,**
**ZENECA Agrochemicals,**
**Jealotts Hill Research Station,**
**P.O. Box 3538**
**Bracknell, Berkshire RG12 6YA (GB)**

(56) References cited:
**EP-A- 0 381 330**
**EP-A- 0 468 682**
**EP-A- 0 468 683**
**EP-A- 0 470 711**
**GB-A- 1 274 282**

- **JOURNAL OF ORGANIC CHEMISTRY. vol. 31 , July 1966 , WASHINGTON DC US pages 2319 - 2321 H.BADER E.A. 'Nucleophilic Displacements of Activated Fluorine in Aromatic Compounds'**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to novel fungicidal acylaminobenzenes, to processes for preparing them, to fungicidal compositions containing them and to methods of using them to combat fungi, especially fungal infections of plants.

Fungicidal acylaminobenzamides are disclosed in EP-A1-038 1330, EP-A1-0468682, EP-A1-0468683 and EP-A1-0470711, whilst benzohydroxamic acid derivatives are disclosed in GB-A-274282.

According to the present invention there is provided a compound of formula (Ia), wherein R' is $C_{1-4}$ alkyl; and R" is $C_{1-4}$ alkoxy.

Alkyl groups, and the alkyl moiety of alkoxy, of R' and R" are straight or branched chains and are, for example, methyl, ethyl or n- or iso-propyl.

The invention is illustrated by the compounds listed in Table I which follows. The compounds have the formula (Ia) in which the values R' and R" are as listed.

TABLE I

| Compound No. | R' | R" | mpt °C |
|---|---|---|---|
| 1 | $CH_3$ | $OC_2H_5$ | 120-5 |
| 2 | $C_2H_5$ | $OCH_3$ | 82.5-84 |
| 3 | $CH_3$ | $OCH_3$ | 101-104 |
| 4 | $C_2H_5$ | $OC_2H_5$ | |
| 5 | $CH_3CH_2CH_2$ | $OCH_3$ | |
| 6 | $CH_3CH_2CH_2$ | $OC_2H_5$ | |
| 7 | $(CH_3)_2CH$ | $OCH_3$ | |
| 8 | $(CH_3)_2CH$ | $OC_2H_5$ | |

The compounds of formula (Ia) can be made by, for example, the methods illustrated in Schemes 1 and 2. Throughout these Schemes R' and R" are as hereinbefore defined. In Scheme 1 compounds of formula (Ia) can be prepared by reacting compounds of formula (VI) with 2-fluoro-iso-butyryl chloride ($F(CH_3)_2CCOCl$) in a suitable organic solvent such as methylene chloride or toluene in the presence of a base such as an amine (for example triethylamine or pyridine) or an alkali metal carbonate, bicarbonate or hydroxide (for example sodium bicarbonate or sodium hydroxide).

Alternatively, compounds of formula (Ia) can be prepared by reacting compounds of formula (VI) with 2-fluoro-iso-butyric acid ($F(CH_3)_2CCO_2H$) in a suitable organic solvent such as dichloromethane or toluene in the presence of a suitable coupling agent (for example a carbodiimide salt such as dimethylaminopropyl ethyl carbodiimide hydrochloride).

Compounds of formula (VI) may be prepared from compounds of formula (XIII), wherein R is lower alkyl (either straight or branched chain) by treatment with an acid (for example aqueous hydrogen chloride, aqueous hydrogen bromide or trifluoroacetic acid) optionally in a solvent such as dichloromethane or tetrahydrofuran.

Compounds of formula (XIII) may be prepared from acids of formula (XII) by reaction with a secondary amine HNR'R" in the presence of a coupling reagent (for example a carbodiimide salt such as dimethylaminopropyl ethyl carbodiimide hydrochloride). A suitable organic solvent may be used such as dichloromethane or toluene.

Compounds of formula (XII) may be prepared by hydrolysis of compounds of formula (XI), wherein R* is, for example, a $C_{1-4}$ alkyl group, by means of aqueous base. Suitable bases include sodium hydroxide or potassium hydroxide. Elevated temperatures may be optionally used (for example the mixture may be heated under reflux).

Compounds of formula (XI) may be prepared from compounds of formula (X) by treatment first with a strong base (for example a butyl lithium) followed by treatment with an alkoxycarbonyl chloride R*OCOCl wherein R* is as defined above. A suitable solvent such as tetrahydrofuran may be used.

Compounds of formula (X) may be prepared from 3,5-difluorobenzoic acid by the Curtius reaction of the corresponding acid azide. 3,5-Difluorobenzoic acid may be treated with an azide (for example diphenylphosphoryl azide) in the presence of an alcohol ROH, wherein R is as defined above, which may be present in excess and thus act as solvent for the reaction. Elevated temperatures may advantageously be used. 3,5-Difluorobenzoic acid is commercially available.

In Scheme 2, compounds of formula (Ia) can be prepared from a compound of formula (IX) by reaction with an amine R'R"NH in a suitable organic solvent such as methylene chloride or tetrahydrofuran in the presence of a base such as triethylamine, sodium bicarbonate or excess R'R"NH.

Acid chlorides of formula (IX) can be prepared from the carboxylic acid of formula (VIII) by reaction with a standard reagent such as oxalyl chloride in a suitable dry solvent such as tetrahydrofuran or methylene chloride and with a catalytic quantity of N,N-dimethylformamide being added if necessary.

The carboxylic acid of formula (VIII) can be prepared from the 4-aminobenzoic acid (VII) by reaction with 2-fluoro-iso-butyryl chloride ($F(CH_3)_2CCOCl$) in the presence of at least two equivalents of a base such as an alkali metal carbonate, bicarbonate or hydroxide (for example sodium bicarbonate) or a tertiary amine (for example pyridine or triethylamine).

The 4-aminobenzoic acid (VII) can be prepared by hydrolysing the compound of formula (XVI) in the presence of water, a suitable base (for example an alkali metal hydroxide such as potassium hydroxide), and optionally in a suitable solvent.

The compound of formula (XVI) can be prepared by the hydrogenation of a compound of formula (XV) (wherein $R^7$ and $R^8$ are, independently, optionally substituted benzyl groups) in the presence of a catalyst (such as a palladium catalyst, for example palladium on charcoal), a solvent (such as an alcohol, for example methanol or ethanol) and an organic acid (for example trifluoroacetic acid).

Optionally substituted benzyl groups are benzyl groups preferably carrying ring substituents selected from the list comprising halogen, $C_{1-4}$ alkyl, $C_{1-4}$ alkoxy, $C_{1-4}$ haloalkyl or $C_{1-4}$ haloalkoxy, but more preferably the benzyl groups are unsubstituted.

Halogen includes chlorine and fluorine.

Compounds of formula (XV) may be prepared by hydrolysis of a compound of formula (XIV) (wherein $R^7$ and $R^8$ are as defined above) in the presence of a base (such as an alkali metal carbonate, for example potassium carbonate) and optionally in the presence of a suitable peroxide (for example hydrogen peroxide) and in a solvent (for example a mixture of dimethylsulphoxide and water).

A compound of formula (XIV) may be prepared by reacting 2,4,6-trifluorobenzonitrile with $R^7R^8NH$ (wherein $R^7$ and $R^8$ are as defined above) in a suitable dipolar aprotic solvent and in the presence of a suitable base (for example triethylamine). Dipolar aprotic solvents are, for example, dimethylsulphoxide, N,N-dimethylformamide, N,N-dimethylacetamide, N,N-diethylacetamide, hexamethylphosphoramide, tetramethylurea, sulpholane, N-methylpyrrolidone, acetonitrile, methylethylketone, 1,3-dimethyl-3,4,5,6-tetrahydro-(2,1H)-pyrimidone (DMPU), dimethylsulphone, N-methyl-hexahydropyridone or N-methyl-ε -caprolactam.

The compounds of formula (Ia) can be prepared using methods and techniques described in EP-A-0381330 and in EP Application No. 91306443.2.

In another aspect, the invention provides processes as herein described for preparing the compounds of the invention.

The compounds of formula (Ia) show fungicidal activity across a range of plant diseases. They are, however, particularly active against the class of pathogens known as the phycomycetes (equivalent to the oomycetes). These include species of Phytophthora, Plasmopara, Peronospora and Pseudoperonospora. Examples of pathogens which the invention compounds are particularly useful for controlling are: Plasmopara viticola on vines; other downy mildews such as Bremia lactucae on lettuce; Peronospora spp. on soybeans, tobacco, onions and other hosts; Pseudoperonospora humuli on hops and Pseudoperonospora cubensis on cucurbits; Phytophthora infestans on potatoes and tomatoes and other Phytophthora spp. on vegetables, strawberries, avocado, pepper, ornamentals, tobacco, cocoa and other hosts; and Pythium sp on rice, horticultural plants, vegetables and turf.

The compounds may move acropetally/locally in plant tissue. Moreover, the compounds may be volatile enough to be active in the vapour phase against fungi on the plant.

The invention therefore provides a method of combating fungi which comprises applying to a plant, to a seed of a plant or to the locus of the plant or seed a fungicidally effective amount of a compound as hereinbefore defined, or a composition containing the same.

The compounds may be used directly for agricultural purposes but are more conveniently formulated into compositions using a carrier or diluent. The invention thus provides fungicidal compositions comprising a compound as hereinbefore defined and an acceptable carrier or diluent therefor. It is preferred that all compositions, both solid and liquid formulations, comprise 0.0001 to 95%, more preferably l to 85%, for example 1 to 25% or 25 to 60%, of a compound as hereinbefore defined.

When applied the foliage of plants, the compounds of the invention are applied at rates of 0.1g to 10Kg, preferably 1g to 8Kg, more preferably 10g to 4Kg, of active ingredient (invention compound) per hectare.

When used as seed dressings, the compounds of the invention are used at rates of 0.0001g (for example 0.001g or 0.05g) to 10g, preferably 0.005g to 8g, more preferably 0.005g to 4g, of active ingredient (invention compound) per kilogram of seed.

The compounds can be applied in a number of ways. For example, they can be applied, formulated or unformulated, directly to the foliage of a plant, to seeds or to other medium in which plants are growing or are to be planted, or they can be sprayed on, dusted on or applied as a cream or paste formulation, or they can be applied as a vapour or as slow release granules.

Application can be to any part of the plant including the foliage, stems, branches or roots, or to soil surrounding the roots, or to the seed before it is planted, or to the soil generally, to paddy water or to hydroponic culture systems.

The invention compounds may also be injected into plants or sprayed onto vegetation using electrodynamic spraying techniques or other low volume methods.

The term "plant" as used herein includes seedlings, bushes and trees. Furthermore, the fungicidal method of the invention includes preventative, protectant, prophylactic, systemic and eradicant treatments.

The compounds are preferably used for agricultural and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example, fillers such as kaolin, bentonite, kieselguhr, dolomite, calcium carbonate, talc, powdered magnesia, fuller's earth, gypsum, diatomaceous earth and china clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example, a mineral oil) for assisting the adhesion of the composition to the seed; alternatively the active ingredient can be formulated for seed dressing purposes using an organic solvent (for example, N-methylpyrrol-idone, propylene glycol or N,N-dimethylformamide). The compositions may also be in the form of wettable powders or water dispersible granules comprising wetting or dispersing agents to facilitate the dispersion in liquids. The powders and granules may also contain fillers and suspending agents.

The compositions may also be in the form of soluble powders or granules, or in the form of solutions in polar solvents.

Soluble powders may be prepared by mixing the active ingredient with a water-soluble salt such as sodium bicarbonate, sodium carbonate, magnesium sulphate or a polysaccharide, and a wetting or dispersing agent to improve water dispersibility/solubility. The mixture may then be ground to a fine powder. Similar compositions may also be granulated to form water-soluble granules. Solutions may be prepared by dissolving the active ingredient in polar solvents such as ketones, alcohols and glycol ethers. These solutions may contain surface active agents to improve water dilution and prevent crystallisation in a spray tank.

Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes, ketones such as cyclohexanone and methylcyclohexanone, chlorinated hydrocarbons such as chlorobenzene and trichlorethane, and alcohols such as benzyl alcohol, furfuryl alcohol, butanol and glycol ethers.

Suspension concentrates of largely insoluble solids may be prepared by ball or bead milling with a dispersing agent with a suspending agent included to stop the solid settling.

Compositions to be used as sprays may be in the form of aerosols wherein the formulation is held in a container under pressure of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds.

Alternatively, the compounds may be used in micro-encapsulated form. They may also be formulated in biodegradable polymeric formulations to obtain a slow, controlled release of the active substance.

By including suitable additives, for example additives for improving the uptake, distribution, adhesive power and resistance to rain on treated surfaces, the different compositions can be better adapted for various utilities. Other additives may be included to improve the biological efficacy of the various formulations. Such additives can be surface active materials to improve the wetting and retention on surfaces treated with the formulation and also the uptake and mobility of the active material, or additionally can include oil based spray additives, for example, certain mineral oil and natural plant oil (such as soya bean and rape seed oil) additives, or blends of them with other adjuvants.

The invention compounds can be used as mixtures with fertilisers (e.g. nitrogen-, potassium- or phosphorus-containing Fertilisers). Compositions comprising only granules of fertiliser incorporating, for example coated with, a compound of formula (Ia) are preferred. Such granules suitably contain up to 25% by weight of the compound. The invention therefore also provides a fertiliser composition comprising a fertiliser and the compound of formula (Ia) or a salt or metal complex thereof

Wettable powders, emulsifiable concentrates and suspension concentrates will normally contain surfactants, e.g. a wetting agent, dispersing agent, emulsifying agent or suspending agent. These agents can be cationic, anionic or non-ionic agents. Suitable cationic agents are quaternary ammonium compounds, for example, cetyltrimethylammonium bromide. Suitable anionic agents are soaps, salts of aliphatic monoesters of sulphuric acid (for example, sodium lauryl sulphate), and salts of sulphonated aromatic compounds (for example, sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, the condensation products of the said partial esters

with ethylene oxide, and the lecithins. Suitable suspending agents are hydrophilic colloids (for example, polyvinylpyrrolidone and sodium carboxymethylcellulose), and swelling clays such as bentonite or attapulgite.

Compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 1-85%, for example 1-25% or 25-60%, by weight of the active ingredient. After dilution to form aqueous preparations, such preparations may contain varying amounts of the active ingredient depending upon the intended purpose, but an aqueous preparation containing 0.0001 to 10%, for example 0.005 to 10%, by weight of active ingredient may be used.

The compositions of this invention may contain other compounds having biological activity, e.g. compounds having similar or complementary fungicidal activity or which possess plant growth regulating, herbicidal or insecticidal activity.

An additional fungicidal compound may be present in the composition of the invention. By including another fungicide, the resulting composition can have a broader spectrum of activity or a greater level of intrinsic activity than the compound of formula (Ia) alone. Further the other fungicide can have a synergistic effect on the fungicidal activity of the compound of formula (Ia). Examples of fungicidal compounds which may be included in the composition of the invention are (RS)-1-aminopropylphosphonic acid, (RS)-4-(4-chlorophenyl)-2-phenyl-2-(1H-1,2,4-triazol-1-ylmethyl) butyronitrile, (Z)-N-but-2-enyloxymethyl-2-chloro-2',6'-diethylacetanilide, 1-(2-cyano-2-methoxyiminoacetyl)-3-ethyl urea, 4-(2,2-difluoro-1,3-benzodioxol-4-yl)pyrrole-3-carbonitrile, 4-bromo-2-cyano-N,N-dimethyl-6-trifluoromethylbenzimidazole-1-sulphonamide, 5-ethyl-5,8-dihydro-8-oxo(1,3)-dioxol-(4,5-g)quinoline-7-carboxylic acid, α-[N-(3-chloro-2,6-xylyl)-2-methoxyacetamido]-γ-butyrolactone, N-(2-methoxy-5-pyridyl)-cyclopropane carboxamide, alanycarb, aldimorph, ampropylfos, anilazine, azaconazole, BAS 490F, benalaxyl, benomyl, biloxazol, binapacryl, bitertanol, blasticidin S, bromuconazole, bupirimate, butenachlor, buthiobate, captafol, captan, carbendazim, carbendazim chlorhydrate, carboxin, chinomethionate, chlorbenzthiazone, chloroneb, chlorothalonil, chlorozolinate, clozylacon, copper containing compounds such as copper oxychloride, copper oxyquinolate, copper sulphate, copper tallate, and Bordeaux mixture, cycloheximide, cymoxanil, cyproconazole, cyprofuram, debacarb, di-2-pyridyl disulphide 1,1'-dioxide, dichlofluanid, dichlone, diclobutrazol, diclomezine, dicloran, didecyl dimethyl ammonium chloride, diethofencarb, difenoconazole, O,O-di-iso-propyl-S-benzyl thiophosphate, dimefluazole, dimetconazole, dimethomorph, dimethirimol, diniconazole, dinocap, dipyrithione, ditalimfos, dithianon, dodemorph, dodine, doguadine, edifenphos, epiconazole, etaconazole, ethirimol, ethoxyquin, ethyl (Z)-N-benzyl-N-([methyl(methylthioethylideneamino-oxycarbonyl)amino]thio)-β-alaninate, etridiazole, fenaminosulph, fenapanil, fenarimol, fenbuconazole, fenfuram, fenpiclonil, fenpropidin, fenpropimorph, fentin acetate, fentin hydroxide, ferbam, ferimzone, fluazinam, fludioxonil, fluoroimide, fluquinconazole, flusilazole, flutolanil, flutriafol, folpet, fuberidazole, furalaxyl, furconazole-cis, guazatine, hexaconazole, hydroxyisoxazole, hymexazole, ICIA5504, imazalil, imibenconazole, ipconazole, iprobenfos, iprodione, isopropanyl butyl carbamate, isoprothiolane, kasugamycin, mancozeb, maneb, mepanipyrim, mepronil, metalaxyl, metconazole, methfuroxam, metiram, metiram-zinc, metsulfovax, myclobutanil, NTN0301, neoasozin, nickel dimethyldithiocarbamate, nitrothal-isopropyl, nuarimol, ofurace, organomercury compounds, oxadixyl, oxolinic acid, oxycarboxin, pefurazoate, penconazole, pencycuron, phenazin oxide, phosetyl-Al, phosphorus acids, phthalide, polyoxin D, polyram, probenazole, prochloraz, procymidone, propamocarb, propamocarb hydrochloride, propiconazole, propineb, propionic acid, prothiocarb, pyracarbolid, pyrazophos, pyrifenox, pyrimethanil, pyroquilon, pyroxyfur, pyrrolnitrin, quaternary ammonium compounds, quinconazole, quinomethionate, quintozene, rabenazole, sodium pentachlorophenate, streptomycin, sulphur, tebuconazole, techlofthalam, tecnazene, tetraconazole, thiabendazole, thicyofen, thifluzamide, 2-(thiocyanomethylthio)benzothiazole, thiophanate-methyl, thiram, timibenconazole, tolclofos-methyl, tolylfluanid, triacetate salt of 1,1'-iminodi (octamethylene)diguanidine, triadimefon, triadimenol, triazbutyl, triazoxide, tricyclazole, tridemorph, triforine, triflumizole, triticonazole, validamycin A, vapam, vinclozolin, XRD-563, zineb and ziram.

The compounds of formula (Ia) can be mixed with soil, peat or other rooting media for the protection of plants against seed-borne, soil-borne or foliar fungal diseases.

The following Examples illustrate the invention. Where they are used in the following examples, HYFLO and DISPERSOL are Trade Names or Trade Marks. Where shown, infrared and NMR data are selective; no attempt has been made to list every absorption in all cases. The following abbreviations are used throughout:

d = doublet     m = multiplet
NMR = nuclear magnetic resonance     s = singlet
brs = broad singlet

EXAMPLE 1

This Example illustrates the preparation of4-(2-fluoro-2-methylpropanoamido)-2,6-difluoro-N-methoxy-N-methylbenzamide (Compound No. 3 of Table I).

Step 1

2,4,6-Trifluorobenzonitrile (80g) was dissolved in dimethylsulphoxide (200ml) and to this was added trimethylamine (140ml, dried over molecular sieve) and dibenzylamine (95ml). The resulting mixture was then heated at 60°C for 3 hours, allowed to cool overnight and then heated at 100°C for a further 32 hours.

After allowing the reaction mixture to cool overnight, it was added to cold water (2000ml), with cooling, over 30 minutes and a solid precipitated. The mixture was stirred for 30 minutes and then extracted with toluene (450ml). The layers were separated and the aqueous phase extracted twice more with toluene (2 x 300ml). The organic phases were combined, washed with water (2 x 300ml) and then evaporated under reduced pressure. The resulting solid was dried in a vacuum desiccator, to leave 4-(N,N-dibenzyl)amino-2,6-difluorobenzonitrile as a brown crystalline solid (157.7g, 92.6% yield).

$^1H$ NMR ($CDCl_3$): δ 4.67(s,4H), 6.27(d,2H), 7.15-7.40(m,10H) ppm.

Step 2

To a stirred solution of4-dibenzylamino-2,6-difluorobenzonitrile (1g, 3mmol) in dimethylsulfoxide (3ml) was added an aqueous solution of potassium carbonate (0.2g in 0.3ml, 1.5mmol). 30% Aqueous hydrogen peroxide (0.5ml) was added dropwise at such a rate so as to keep the reaction temperature below 25°C. After 30 minutes, further dimethyl sulfoxide (1ml) and 30% aqueous hydrogen peroxide (0.5ml) were added and the reaction heated to 80°C and kept at this temperature for 2.5 hours. The reaction was quenched by pouring into excess water and extracted three times with ethyl acetate. The combined organic extracts were washed twice with water, dried (magnesium sulphate) and concentrated under reduced pressure to give an oil (1.05g) which crystallised on trituration with diethyl ether. Recrystallisation from hexane/ethyl acetate gave 4-dibenzylamino-2,6-difluorobenzamide (230mg) as off-white crystals. Product from a further preparation had the following physical data: $^1H$ NMR ($CDCl_3$): δ 4.64(s,4H), 5.86(brs, 1H), 6.06(brs, 1H), 6.25(d,2H), 7.15-7.40(m,10H) ppm.

Step 3

5% Palladium on charcoal catalyst (9g) was charged to a 600ml hydrogenation vessel fitted with a cooling coil followed by 4-(N,N-dibenzyl)amino-2,6-difluorobenzamide (60g), methanol (360g) and trifluoroacetic acid (38ml). [Warning: It was found that, if warm, the vessel had to be completely free of methanol vapours to avoid igniting the catalyst.] The vessel was sealed, pressure tested with nitrogen, vented and then pressurised with hydrogen to 20 bar. The stirrer was started and the mixture heated to 50°C. Hydrogen uptake began at 47°C. Heating was switched off 21/2 hours after hydrogen uptake began, the stirrer was switched off ½ hour after that and the mixture was left overnight. After venting and purging with nitrogen (three times), the cooling water was turned off and the mixture reheated to 50°C at atmosphere pressure and kept at this temperature for 1 hour to ensure that the product was in solution.

The catalyst was filtered on to HYFLO and the filter bed washed with copious quantities of methanol. The filtrate was evaporated under reduced pressure to dryness to leave 4-amino-2,6-difluorobenzamide as a brown solid (30.14g). $^1H$ NMR ($d^6$ DMSO) : δ 5.9(brs,2H), 6.10(d,2H), 7.36(brs,1H), 7.57(brs,1H) ppm.

Step 4

To an aqueous solution of potassium hydroxide (257ml of a solution having a molarity between 4.55M and 5.4M) was added 4-amino-2,6-difluorobenzamide (90g) and the resulting mixture was stirred at 100-105°C for 3½ hours and then allowed to cool overnight. Thin layer chromatographic analysis showed the reaction to have gone to completion. The mixture was filtered through HYFLO and the residue was washed with water (2 x 25ml). The filtrate acidified with concentrated hydrochloric acid and the resulting mixture was extracted with ethyl acetate. The ethyl acetate extracts were combined, dried over magnesium sulphate and evaporated in vacuo to leave 4-amino-2,6-difluorobenzoic acid as a buff solid (53.7g). $^1H$ NMR ($d^6$ DMSO) : 6.14(d,2H), 6.29(brs,2H), 12.6(brs,1H) ppm.

Step 5

Reaction carried out under nitrogen.

A mixture of oxalyl chloride (0.49g), N,N-dimethylformamide (a few drops) and 2-fluoro-iso-butyric acid (0.48g) in dry dichloromethane (4ml) was stirred for 1½ hours. The end point of the reaction was gauged by the absence of bubbles when a trace of N,N-dimethylformamide was added to the reaction mixture. The resulting solution was added to a mixture of4-amino-2,6-difluorobenzoic acid (0.67g) and pyridine (1.33ml) in dry dichloromethane (10ml) at 0°C.

After allowing the resulting mixture to stand overnight it was diluted with ethyl acetate (100ml) and then washed

with 2N hydrochloric acid (3 times). The organic solution was dried over anhydrous magnesium sulphate, and evaporated under reduced pressure to leave 2,6-difluoro-4-(2-fluoro-2-methylpropanamido)benzoic acid as a cream solid (0.807g, 80%) which became pink on standing. $^1H$ NMR ($d^6$ DMSO) : $\delta$ 1.52(d,6H), 7.56(d,2H), 10.47(d,1H), 13.59 (brs,1H) ppm.

Step 6

Oxalyl chloride (340 µl) was added in portions to a mixture of 2,6-difluoro-4-(2-fluoro-2-methylpropanamido)benzoic acid (1g) and N,N-dimethylformamide (a few drops) in dry dichloromethane (15ml). The end point of this reaction was gauged in the same way as for step 5.

The resulting mixture was added in portions to a mixture of N,O-dimethylhydroxylamine hydrochloride (560 mg), 4-N,N-dimethylaminopyridine (trace) and triethylamine (2ml) in dry dichloromethane (10ml) at 0°C. The reaction mixture was allowed to stand overnight after which it was washed with 5% aqueous hydrochloric acid and saturated aqueous sodium bicarbonate solution. The resulting organic layer was dried over anhydrous magnesium sulphate and evaporated under reduced pressure to leave a brown solid. This was purified by flash chromatography on silica, eluting with hexane : ethyl acetate 2:1, to give the title compound (750mg) as a colourless oil which, on addition of diethyl ether, crystallised (m.pt. 101-104°C).

EXAMPLE 2

This Example describes the preparation of 4-(2-fluoro-2-methylpropanoamido)-2,6-difluoro-N-methoxy-N-ethyl benzamide (Compound No. 2 Table I).

Step 1

To a suspension of 2,6-difluoro-4-(2-fluoro-2-methylpropanoamido)benzoic acid (500mg, for preparation see Example 1 of UK Application No. 9213568.0) in dichloromethane (10ml) was added, portionwise, oxalyl chloride (200µl) and N,N-dimethylformamide (a few drops). The resultant clear, yellow solution was stirred for one hour and then added, in portions over one hour, to a cooled mixture of O-methylhydroxylamine hydrochloride (240mg), pyridine (1ml) and dichloromethane (10ml). The resulting mixture was allowed to stand overnight after which it was evaporated under reduced pressure. The residue was partitioned between ethyl acetate and 5% aqueous hydrochloric acid. The organic layer was then washed with further 5% aqueous hydrochloric acid, with saturated aqueous sodium bicarbonate solution and was then dried over magnesium sulphate. Evaporation under reduced pressure left 4-(2-fluoro-2-methylpropanoamido)-2,6-difluoro-N-methoxybenzamide as an off-white solid (390mg, 70%) m.p. 180.5-183.5°C.

Step 2

To a stirred mixture of 4-(2-fluoro-2-methylpropanoamido)-2,6-difluoro-N-methoxybenzamide (315mg), potassium carbonate (75mg) and acetone (7.5ml) was added ethyl iodide (90µl). Gas chromatographic analysis of the resulting mixture showed that there was still unreacted starting material and so a further portion of ethyl iodide (500µl) was added to the reaction mixture. The mixture was then refluxed for about 60 hours.
After cooling, the reaction mixture was poured into water and extracted with ethyl acetate. The organic extracts were combined, dried over magnesium sulphate and then evaporated under reduced pressure to leave a yellow gum (390mg). The gum was chromatographed on silica eluting with hexane: ethyl acetate 2:1 to give 4-(2-fluoro-2-methylpropanoamido)-2,6--difluoro-N-methoxy-N-ethylbenzamide as a solid (135mg) and ethyl 4-(2-fluoro-2-methylpropanoamido)-2,6-difluoro-O-methylbenzenecarbohydroximate as a yellow oil (55mg).

EXAMPLE 3

This Example illustrates the preparation of 4-(2-fluoro-2-methylpropanoamido)-2,6-difluoro-N-ethoxy-N-methylbenzaniide (Compound No. 1 of Table I).

Step 1

To a suspension of 2,6-difluoro-4-(2-fluoro-2-methyl-propanoamido)benzoic acid (1.05g) in dry dichloromethane (20ml) was added, in portions, oxalyl chloride (390µl) and N,N-dimethylformamide (a few drops). The resulting solution was added to a cooled mixture of O-ethylhydroxylamine hydrochloride (590mg), pyridine (2ml) and dry dichloromethane (20ml) over 1 hour.

The resulting clear solution was washed with 5% aqueous hydrochloric acid causing separation of a solid. Sufficient ethyl acetate was added to redissolve the solid and the organic layer was separated, and washed with aqueous hydrochloric acid and then with saturated aqueous sodium bicarbonate. The organic layer was then dried over magnesium sulphate and evaporated under reduced pressure to leave 4-(2-fluoro-2-methylpropanoamido)-2,6-difluoro-N-ethoxybenzamide an off-white solid (1.05g, 86%) m.p. 179-180°C.

Step 2

To a refluxing mixture of4-(2-fluoro-2-methylpropanoamido)-2,6-difluoro-N-ethoxybenzamide (500mg), potassium carbonate (115mg) and acetone (10ml) was added methyl iodide (100µl). The resulting mixture was refluxed overnight after which time further methyl iodide (80µl) was added over 4 hours. Further potassium carbonate (15mg) was then added.

The reaction mixture was then partitioned between water and ethyl acetate, the organic layer was separated, dried over magnesium sulphate and evaporated under reduced pressue to leave a yellow gum which crystallised on standing. The gum was chromatographed on silica eluting with hexane:ethyl acetate 2:1 to give 4-(2-fluoro-2-methylpropanoamido)2,6-difluoro-N-ethoxy-N-methylbenzamide as a white solid (370mg) and methyl 4-(2-fluoro-2-methylpropanoamido)-2,6-difluoro-O-ethyl-benzenecarbohydroximate as a yellow oil (48mg).

EXAMPLE 4

This Example illustrates the preparation of4-(2-fluoro-2-methylpropanoamido)-2,6-difluoro-N-methoxy-N-methylbenzamide (Compound No. 3 of Table I).

Methyl iodide (80µl) was added to a stirred mixture of 4-(2-fluoro-2-methylpropanoamido)-2,6-difluoro-N-methoxybenzamide (380mg, for preparation see Example 2), potassium carbonate (90mg) and acetone (10ml). After two hours a further portion of methyl iodide (20µl) was added.

After two hours the reaction mixture was poured onto water and extracted with ethyl acetate. The extracts were combined, washed with water, dried over magnesium sulphate and evaporated under reduced pressure to leave a pale yellow gum (290mg). The gum was chromatographed on silica, eluting with hexane:ethyl acetate (2:1) to give 4-(2-fluoro-2methylpropanoamido)-2,6-difluoro-N-methoxy-N-methylbenzamide and methyl 4-(2-fluoro-2-methylpropanoamido-2,6-difluoro-O-methylbenzenecarbohydroximate (8mg) as an oil.

The following are examples of compositions suitable for agricultural and horticultural purposes which can be formulated from the compounds of the invention. Such compositions form another aspect of the invention. Percentages are by weight.

EXAMPLE 5

An emulsifiable concentrate is made up by mixing and stirring the ingredients until all are dissolved.

| | |
|---|---|
| Compound No. 3 of Table I | 10% |
| Benzyl alcohol | 30% |
| Calcium dodecylbenzenesulphonate | 5% |
| Nonylphenolethoxylate (13 mole ethylene oxide) | 10% |
| Alkyl benzenes | 45% |

EXAMPLE 6

The active ingredient is dissolved in methylene dichloride and the resultant liquid sprayed on to the granules of attapulgite clay. The solvent is then allowed to evaporate to produce a granular composition.

| | |
|---|---|
| Compound No. 3 of Table I | 5% |
| Attapulgite granules | 95% |

EXAMPLE 7

A composition suitable for use as a seed dressing is prepared by grinding and mixing the three ingredients.

| | |
|---|---|
| Compound No. 3 of Table I | 50% |
| Mineral oil | 2% |
| China clay | 48% |

EXAMPLE 8

A dustable powder is prepared by grinding and mixing the active ingredient with talc.

| | |
|---|---|
| Compound No. 3 of Table I | 5% |
| Talc | 95% |

EXAMPLE 9

A suspension concentrate is prepared by ball milling the ingredients to form an aqueous suspension of the ground mixture with water.

| | |
|---|---|
| Compound No. 3 of Table I | 40% |
| Sodium lignosulphonate | 10% |
| Bentonite clay | 1% |
| Water | 49% |

This formulation can be used as a spray by diluting into water or applied directly to seed.

EXAMPLE 10

A wettable powder formulation is made by mixing together and grinding the ingredients until all are thoroughly mixed.

| | |
|---|---|
| Compound No. 3 of Table I | 25% |
| Sodium lauryl sulphate | 2% |
| Sodium lignosulphonate | 5% |
| Silica | 25% |
| China clay | 43% |

EXAMPLE 11

A soluble powder is made by mixing and grinding the ingredients to form an homogeneous powder.

| | |
|---|---|
| Compound No. 3 of Table I | 10% |
| Sodium dioctylsulphosuccinate | 2% |
| Sodium lignosulphonate | 5% |
| Sodium benzoate | 20% |
| Sodium bicarbonate | 63% |

EXAMPLE 12

A soluble granule is made by adding 10-20% water to the soluble powder composition prepared as described in Example 11 to form damp granules, which are then dried.

EXAMPLE 13

A water soluble concentrate is made by mixing together and dissolving the active ingredient in the other ingredients.

| | |
|---|---|
| Compound No. 3 of Table I | 10% |
| $C_{13/15}$ alcohol/7 mole ethylene oxide | 5% |
| Propylene glycol monomethyl ether | 85% |

EXAMPLE 14

The compounds of Table I were tested against the diseases Plasmopora viticola on vine and Phytophthora infestans lycopersici on tomato. The technique employed was as follows.

The plants were grown in John Innes Potting Compost (No. 1 or 2) in 4cm diameter minipots. The test compounds were formulated either by bead milling with aqueous DISPERSOL T or as a solution in acetone or acetone/ethanol which was diluted to the required concentration immediately before use. The formulations (100 ppm active ingredient) were sprayed onto the foliage or applied to the roots of the plants in the soil. The sprays were applied to maximum retention and the root drenches to a final concentration equivalent to approximately 40 ppm a.i. in dry soil.

For most of the tests the compounds were applied to the soil (roots) or to the foliage (by spraying) one or two days before the plant was inoculated with the disease. The pathogens were applied by spray as spore suspensions onto the leaves of test plants. After inoculation, the plants were put into an appropriate environment to allow infection to proceed and then incubated until the disease was ready for assessment. The period between inoculation and assessment varied from four to seven days according to the disease and environment.

The disease control was assessed by visual assessment of the percentage leaf area covered by actively sporulating disease. Assessments were performed on a single leaf of each of the two replicate plants, and the mean value of these two recordings was calculated for each treatment. The mean value for each treatment was then expressed as a percentage of the level of disease present on the untreated control plants. This calculated value is referred to as a POCO (Percentage of Control) value. An example of a typical calculation is as follows:

Mean disease level on untreated Control = 90
Mean disease level on treatment A = 30

$$\text{POCO for treatment A} = \frac{\text{Mean disease level on treatment A}}{\text{Mean disease level on untreated Control}} \times 100$$

$$= \frac{30}{90} \times 100 = 33.3$$

Thus a POCO value of 0 indicates complete disease control.

The results are shown in Table II.

TABLE II

| Compound No | Pv syst | Pv prot | Pil syst | Pil prot |
|---|---|---|---|---|
| 1 | 0 | 61 | 4 | 50 |
| 2 | 0 | 13 | 0 | 100 |
| 3 | 0 | 0 | 0 | 67 |

Pv = Plasmopara viticola
Pil = Phytophthora infestans lycopersici
syst = root drench
prot = foliar spray

CHEMICAL FORMULAE

(IN DESCRIPTION)

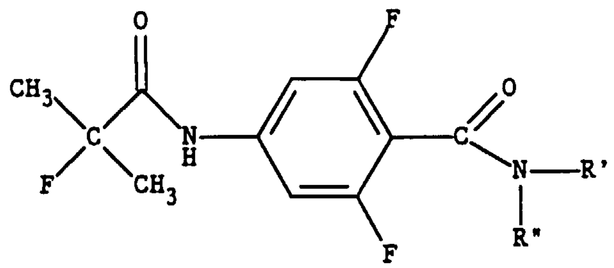

(Ia)

Scheme 1

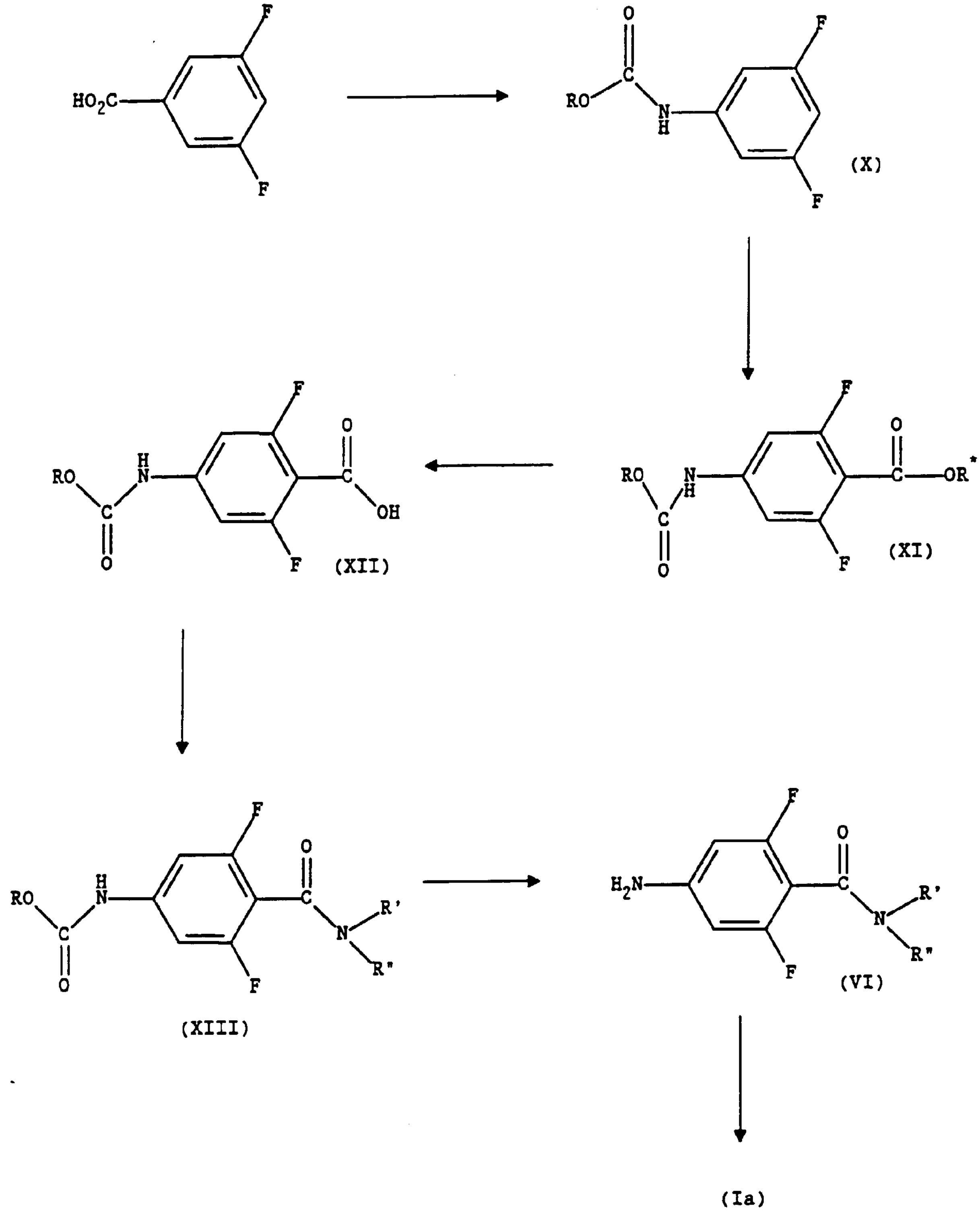

(X) (XI) (XII) (XIII) (VI) (Ia)

Scheme 2

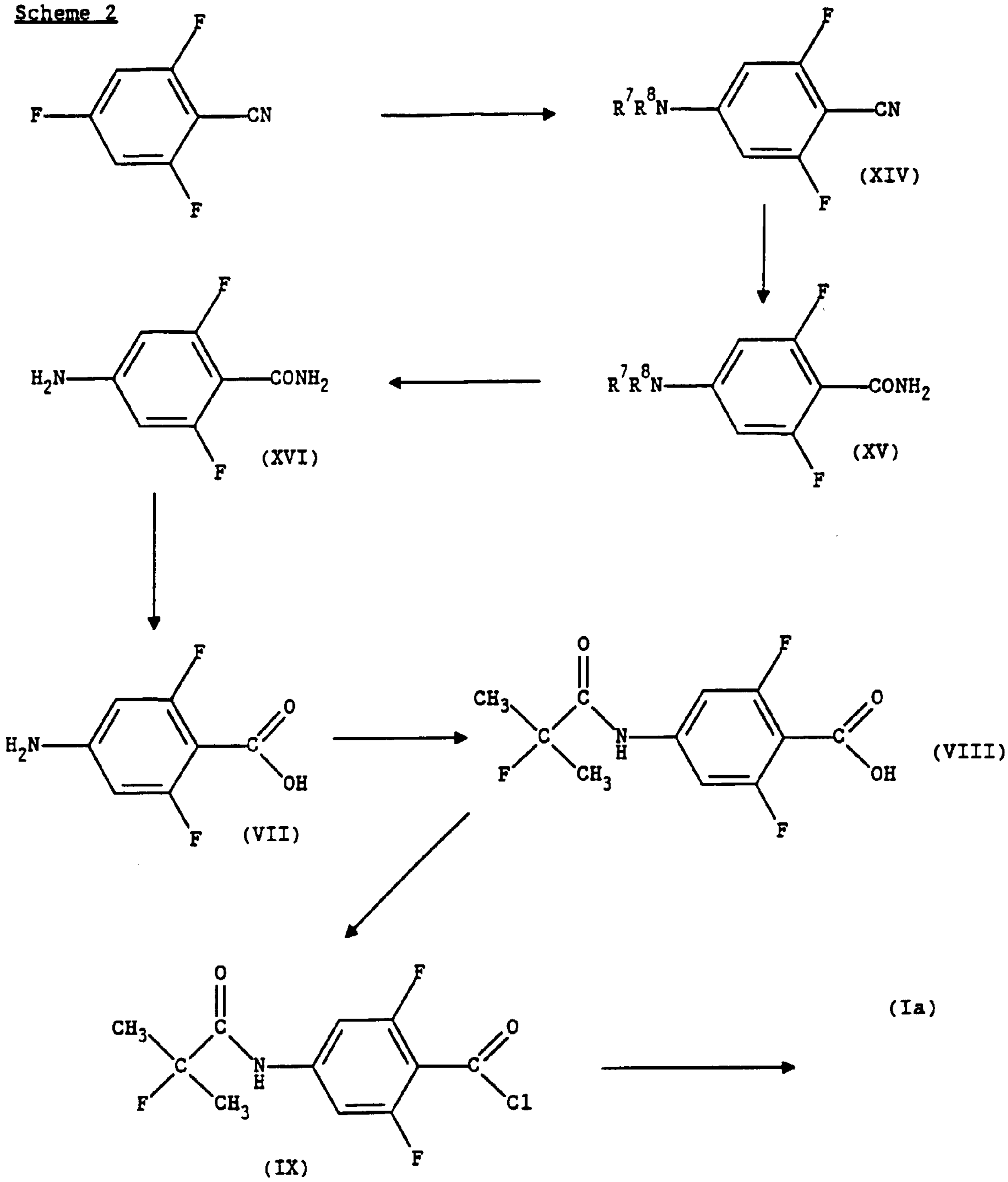

(XIV)

(XV)

(XVI)

(VII)

(VIII)

(IX)

(Ia)

## Claims

1. A compound of the formula (Ia):

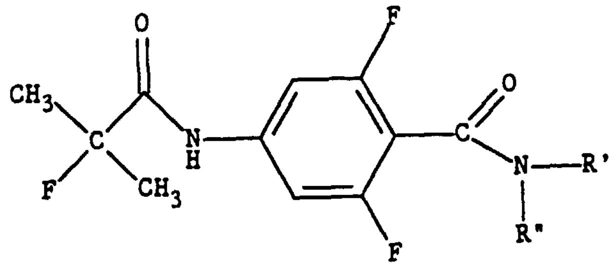

(Ia)

in which R' is $C_{1-4}$ alkyl, and R" is $C_{1-4}$ alkoxy.

**2.** A compound as claimed in claim 1 wherein R' is methyl and R" is $C_{1-4}$ alkoxy.

**3.** A compound as claimed in claim 1 wherein R' is methyl and R" is methoxy.

**4.** A fungicidal composition comprising a fungicidally effective amount of a compound according to claim 1 and a fungicidally acceptable carrier or diluent therefor.

**5.** A method of combating fungi which comprises applying to plants, to the seeds of plants or to the locus of the plants or seeds, a compound according to claim 1 or a composition according to claim 4.

**6.** A process for preparing a compound as claimed in claim 1 comprising:

i) reacting a compound of formula (VI):

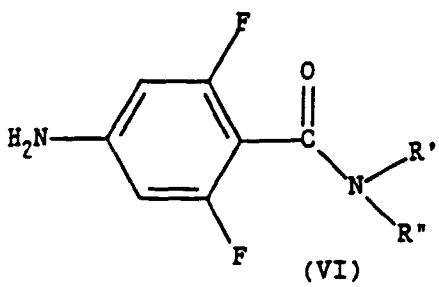

(VI)

with either $F(CH_3)_2CCOCl$, in a solvent and in the presence of a base, or $F(CH_3)_2CCO_2H$ in a solvent and in the presence of a coupling agent; or

ii) reacting the compound of formula (IX):

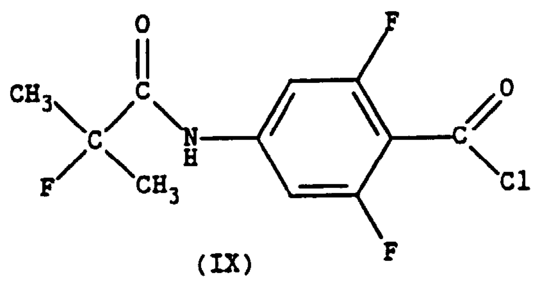

(IX)

with an amine R'R"NH in a solvent and in the presence of a base or an excess of R'R"NH.

**Patentansprüche**

1. Verbindung der Formel (Ia):

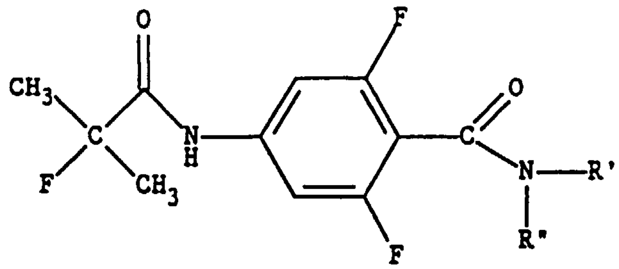

(Ia)

in der R' $C_{1-4}$-Alkyl und R" $C_{1-4}$-Alkoxy ist.

2. Verbindung nach Anspruch 1, wobei R' Methyl und R" $C_{1-4}$-Alkoxy ist.

3. Verbindung nach Anspruch 1, wobei R' Methyl und R" Methoxy ist.

4. Fungicide Zusammensetzung, die eine fungicid wirksame Menge einer Verbindung nach Anspruch 1 und einen für Fungicide akzeptablen Träger oder Verdünnungsmittel enthält.

5. Verfahren zur Bekämpfung von Pilzen, bei dem auf Pflanzen, auf das Saatgut von Pflanzen oder auf den Standort der Pflanzen oder des Saatguts eine Verbindung nach Anspruch 1 oder eine Zusammensetzung nach Anspruch 4 aufgebracht wird.

6. Verfahren zur Herstellung einer Verbindung nach Anspruch 1, bei dem:

   i) eine Verbindung der Formel (VI):

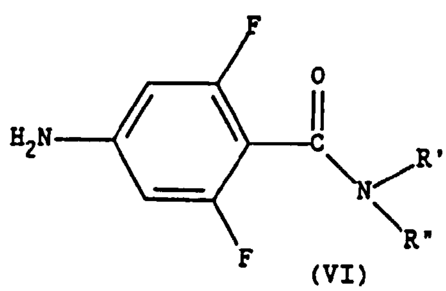

(VI)

   entweder mit $F(CH_3)_2CCOCl$ in einem Lösungsmittel und in Gegenwart einer Base, oder mit $F(CH_3)_2CCO_2H$ in einem Lösungsmittel und in Gegenwart eines Kupplungsmittels umgesetzt wird, oder

   ii) ein Verbindung der Formel (IX):

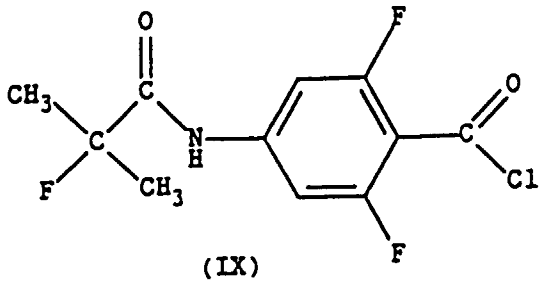

(IX)

mit einem Amin R'R"NH in einem Lösungsmittel und in Gegenwart einer Base oder eines Überschusses an R'R"NH umgesetzt wird.

## Revendications

1. Composé de formule (Ia)

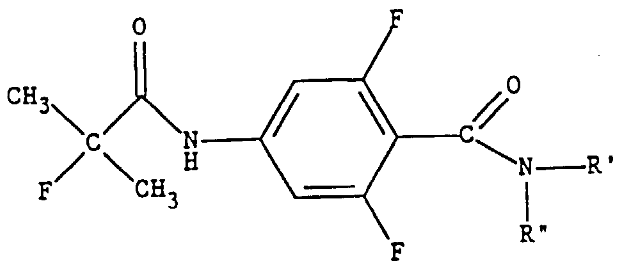

(Ia)

dans laquelle R' représente un groupe alkyle en $C_1$ à $C_4$, et R" représente un groupe alkoxy en $C_1$ à $C_4$.

2. Composé suivant la revendication 1, dans lequel R' représente un groupe méthyle et R" représente un groupe alkoxy en $C_1$ à $C_4$.

3. Composé suivant la revendication 1, dans lequel R' représente un groupe méthyle et R" représente un groupe méthoxy.

4. Composition fongicide comprenant une quantité, efficace du point de vue fongicide, d'un composé suivant la revendication 1 et un support ou diluant acceptable du point de vue fongicide.

5. Procédé pour lutter contre des champignons, qui comprend l'application à des plantes, aux graines de plantes ou au milieu dans lequel se trouvent les plantes ou les graines, un composé suivant la revendication 1 ou une composition suivant la revendication 4.

6. Procédé pour la préparation d'un composé suivant la revendication 1, comprenant :

   i) la réaction d'un composé de formule (VI)

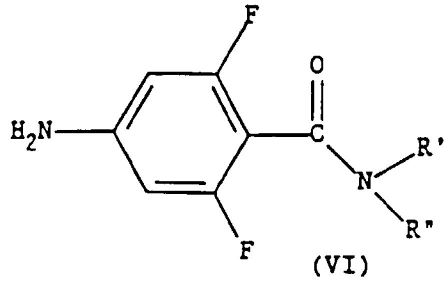

(VI)

avec un composé de formule $F(CH_3)_2CCOCl$, dans un solvant et en présence d'une base, ou bien avec un composé de formule $F(CH_3)_2CCO_2H$ dans un solvant et en présence d'un agent de couplage ; ou

ii) la réaction du composé de formule (IX) :

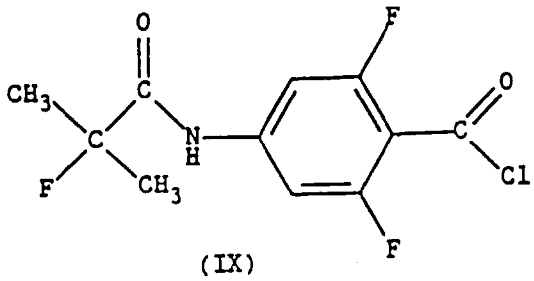

(IX)

avec une amine de formule R'R"NH dans un solvant et en présence d'une base ou d'un excès d'amine de formule R'R"NH.